# EUROPEAN PATENT SPECIFICATION

(11) **EP 3 881 826 B1**
(45) Date of publication and mention of the grant of the patent: **22.04.2026**
(21) Application number: 21161059.7
(22) Date of filing: 05.03.2021
(51) Int. Cl.: A61K 9/00, A61K 47/44, A61K 31/00

(54) **NATURAL NOSE AND THROAT SPRAY**
NATÜRLICHES NASEN- UND RACHENSPRAY
SPRAY NATUREL POUR LE NEZ ET LA GORGE

(30) Priority: 16.03.2020 IT 202000005539
(43) Date of publication of application: 22.09.2021
(73) Proprietor: MULTIOSSIGEN S.P.A., 24020 Gorle (BG) (IT)
(72) Inventor: FRANZINI, Marianno, 24129 Bergamo (BG) (IT)
(74) Representative: Gatti, Enrico

(56) References cited:
- WO-A1-2015/122999
- WO-A2-2019/240713
- IT-A1- UB20 155 024
- RU-A- 2003 121 747
- RU-A- 2010 151 345

## Description

The present invention relates to a mixture for a natural nose and throat spray, in particular comprising ozone.

Ozone is a powerful oxidant, has antibacterial activity and performs antalgic and anti-inflammatory activities.

Several prior art documents relating to this technical field are known, such as WO 2019/240713 A2, IT UB2015 5024 A1, RU 2010 151345 A, RU 2003 121747 A, and WO 2015/122999 A1.

For example, WO 2019/240713 A2 describes a solution comprising ozonized oil in nano or micron sizes, water, an emulsifier, and 10-100,000 mg/L of ozone gas.

The aim of the present invention is to provide a mixture for a nose and throat spray that is natural.

A further aim is to provide a nasal decongestant spray.

In accordance with the present invention, these aims and others still are achieved by the attached claim**.**

The advantages of this solution compared to the solutions of the known art are numerous.

The natural nose and throat spray, in accordance with the present invention, is used to moisten the nasal turbinates in the case of allergic or vasomotor rhinitis.

Rhinitis is a medical term that describes irritation and inflammation of the nasal mucosa.

Thanks to the presence of ozone in the nasal spray, irritation and inflammation of the nasal mucosa is eliminated.

The characteristics and the advantages of the present invention will become clear from the following detailed description of a practical embodiment thereof, described as a non-limiting example.

A mixture for a natural nose and throat spray, in accordance with the present invention, consisting of a combination of water from 97% to 99% of the overall volume and of ozonated vegetable oil from 3% to 1% of the overall volume. With no other addition.

The doses of ozone in the water are zero.

1 to 2 ppm of ozone are present in 1 litre of ozonated vegetable oil.

The water used is distilled water or deionized water.

The oil used is an edible vegetable oil, for example, sunflower oil. However, it is also possible to use corn oil, peanut oil, mixed seed oil.

In order to ozonate the vegetable oil, an ozone generator is used that produces, in a known manner, from 1 to 100 g/hour of ozone which is fed into a mixing chamber with the oil, so as to obtain an ozone concentration of 4-10 mg/l, a mixing pressure of 0.1 to 6 bar, and is kept in the chamber for a time comprised between 1 and 6 hours.

The composition thus obtained is inserted in a spray container without propellants, i.e., a nebulizer that transforms a liquid into an aerosol having very fine droplets, in order to keep the spray completely natural.

The spray container must be shaken before use to mix the product better.

The product is shaken, so there is no need to use emulsifiers that could alter the effectiveness of the product.

The ozone is only added to the oil so that it can be preserved for a longer time compared to in the water.

Moreover, by adding the ozone to the oil, ozonides are created and therefore there is a greater retention of the same within the oil.

In fact, the ozone is maintained within the oil for up to around two years with only a few percent loss (1% to 3%) of the ozone content. This unlike water which retains the ozone for only a few tens of minutes, especially at higher temperatures.

## Claims

1. A method for providing a mixture for a natural nose and throat spray, the mixture consisting of a combination of water and ozonated vegetable oil, said method comprising in the steps of mixing 97% to 99% of water of the overall volume; with 3% to 1% of ozonated vegetable oil of the overall volume; said ozonated vegetable oil is produced by introducing it into a mixing chamber with ozone produced by an ozone generator that produces from 1 to 100 g/hour of ozone in order to obtain an ozone concentration of 4 to 10 mg/l, with a mixing pressure from 0.1 10⁵ Pa to 6 10⁵ Pa (0.1 to 6 bar) for a time of 1 to 6 hours; introducing said mixture in a nebulizer without propellants; and shaken said nebulizer before use.

## Patentansprüche

1. Verfahren zur Bereitstellung einer Mischung für ein natürliches Nasen und Rachenspray, wobei die Mischung aus einer Kombination aus Wasser und ozonisiertem pflanzlichem Öl besteht, wobei das Verfahren die folgenden Schritte umfasst: Mischen von 97% bis 99% Wasser des Gesamtvolumens mit 3% bis 1% ozonisiertem pflanzlichem Öl des Gesamtvolumens; wobei das ozonisierte pflanzliche Öl dadurch hergestellt wird, dass es in eine Mischkammer mit Ozon eingebracht wird, das von einem Ozongenerator erzeugt wird, der 1 bis 100 g/Stunde Ozon erzeugt, um eine Ozonkonzentration von 4 bis 10 mg/l zu erhalten, bei einem Mischdruck von 0,1 10⁵ Pa bis 6 10⁵ Pa (0,1 bis 6 bar) für eine Zeit von 1 bis 6 Stunden; Einbringen der genannten Mischung in einen Zerstäuber ohne Treibmittel; und Schütteln des genannten Zerstäubers vor der Verwendung.

## Revendications

1. Procédé pour fournir un mélange destiné à un spray nasal et pour la gorge naturel, le mélange consistant en une combinaison d'eau et d'huile végétale ozonée, ledit procédé comprenant les étapes consistant à: mélanger 97% à 99% d'eau du volume total avec 3% à 1% d'huile végétale ozonée du volume total; ladite huile végétale ozonée étant produite en l'introduisant dans une chambre de mélange avec de l'ozone produit par un générateur d'ozone qui produit de 1 à 100 g/heure d'ozone afin d'obtenir une concentration d'ozone de 4 à 10 mg/l, avec une pression de mélange de 0,1 10⁵ Pa à 6 10⁵ Pa (0,1 à 6 bar) pendant un temps de 1 à 6 heures; introduire ledit mélange dans un nébuliseur sans propulseurs; et agiter ledit nébuliseur avant utilisation.
